(19) Europäisches Patentamt

European Patent Office

Office européen des brevets

(11) Veröffentlichungsnummer: **0 242 535 B1**

(12) **EUROPÄISCHE PATENTSCHRIFT**

(45) Veröffentlichungstag der Patentschrift: **23.09.92**

(51) Int. Cl.⁵: **C07D 213/80**, C07D 213/803

(21) Anmeldenummer: **87102566.4**

(22) Anmeldetag: **24.02.87**

Die Akte enthält technische Angaben, die nach dem Eingang der Anmeldung eingereicht wurden und die nicht in dieser Patentschrift enthalten sind.

(54) **Verfahren zur Herstellung grobkristalliner Nicotinsäure hoher Reinheit.**

(30) Priorität: **25.04.86 DE 3614019**

(43) Veröffentlichungstag der Anmeldung:
**28.10.87 Patentblatt 87/44**

(45) Bekanntmachung des Hinweises auf die Patenterteilung:
**23.09.92 Patentblatt 92/39**

(84) Benannte Vertragsstaaten:
**AT BE CH DE ES GB IT LI NL SE**

(56) Entgegenhaltungen:
**DE-A- 6 129**
**DE-C- 828 246**
**GB-A- 1 474 461**

**HOUBEN-WEYL "Methoden der Organischen Chemie", Band E 5, 1985, Seiten 263-265, Georg Thieme Verlag, Stuttgart; R. SUSTMANN et al.: "I. Carbonsäuren, deren Salze sowie Aminosäuren"**

**CHEMICAL ABSTRACTS, Band 65, Nr. 4, 15. August 1966, Spalte 446b, Columbus, Ohio, US**

**CHEMICAL ABSTRACTS, Band 38, Nr. 1, 10.**

**Januar 1944, Spalte 1006, Columbus, Ohio, US; C.F. KREWSON et al.: "Hydrolysis of nicotinonitrile by ammonia"**

**ENCYCLOPEDIA OF CHEMICAL TECHNOLOGY, 3. Ausgabe, Band 24, 1984, Seiten 59-93, John Wiley & Sons, New York, US; H. OFFERMANS et al.: "Nicotinamide and nicotinic acid"**

(73) Patentinhaber: **Degussa Aktiengesellschaft**
**Weissfrauenstrasse 9**
**W-6000 Frankturt am Main 1(DE)**

(72) Erfinder: **Möller, Alexander, Dr.**
**Steinesweg 7**
**W-6464 Linsengericht 4(DE)**
Erfinder: **Friedrich, Heinz, Dr.**
**Grünaustrasse 4**
**W-6450 Hanau 9(DE)**
Erfinder: **Kuhn, Herbert, Dr.**
**Berliner Strasse 15**
**W-8755 Alzenau(DE)**
Erfinder: **Winkler, Kurt**
**Ringstrasse 8**
**W-6365 Rossbach v.d. H.(DE)**

## Beschreibung

Die vorliegende Erfindung betrifft ein Verfahren zur Herstellung grobkristalliner Nicotinsäure hoher Reinheit, die man aus alkalischen Alkalinicotinat-Lösungen durch Zusatz von Mineralsäure ausfällt.

Zur Herstellung von Nicotinsäure sind viele Verfahren bekannt.

Man kann sie durch Flüssigphasenoxidation von Alkylpyridinen (DE-A-2 046 556, US-A-4 001 257), saure Hydrolyse des 3-Cyanopyridins (DE-A-2 438 263), oder Verseifung von 3-Cyanopyridin mit Ammoniak und anschließender Zersetzung des Ammoniumnicotinats mittels Wasserdampfdestillation gewinnen (US-A-3 929 811). C.A. 38 (1944) Spalte 100,8 betrifft ebenso die Hydrolyse mit Ammoniak, die gegebenenfalls durch NaOH beschlennigt wird.

Bekannt ist die Verwendung von Natronlauge für diesen Zweck auch aus C.A. 65, 5446 a (1966), PL-A-50 077, die Autoren dieser Patentschrift halten jedoch Ammoniak für geeigneter.

Gemäß Houben-Weyl, Band E5 (1985), seite 265, setzt man für die Hydrolyse von 3-Cyanopyridin ethanolische NaOH ein.

Die DE-C-828 246 betrifft auch die Verseifung von 3-Cyanopyridin. Gemäß dieser Druckschrift setzt man das 3-Cyanopyridin mit einem Überschuß an Bariumhydroxid um, fällt anschließend das Barium mit Schwefelsäure aus und trennt eine Rohnicotinsäure ab, die noch gereinigt werden muß.

Die geringe Selektivität hat bisher die technische Realisierung der katalytischen Gasphasenreaktion verhindert.

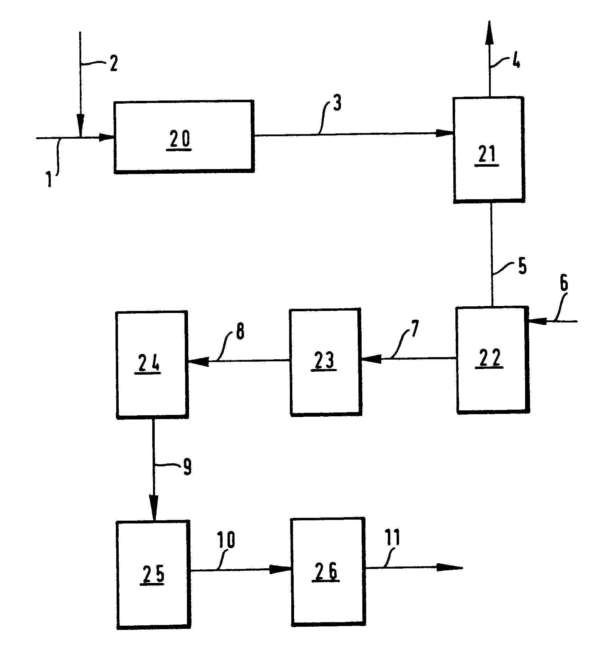

Die Verfahren, die durch ammoniokalische Verseifung von 3-Cyanopyridin Nicotinsäure gewinnen, besitzen den Nachteil, daß das hier anfallende Nebenprodukt Nicotinsäureamid Selektivität und Ausbeute schmälert und außerdem hohe Reaktionszeiten nötig sind (J.E. Paustian et al. Chemtech March 1981, 176 Fig. 4. J.A. Arkhipova et al. Zurnal Prikl. Khim. 35 (2), 366-369, (1962), B.V. Suvorov et al. Zurnal Prikl. Khim. 45, 2716-2718 (1972)). Wegen der starken Sublimationsneigung der Nicotinsäure führt die thermische Zersetzung von Ammoniumnicotinat mittels Wasserdampf zu Betriebsstörungen infolge von Verstopfungen, die sich nur durch hohen apparativen Aufwand minimieren lassen (DE-A-2 423 755).

Ein Nachteil aller oben beschriebenen Verfahren besteht darin, daß man eine Nicotinsäure erhält, die erst durch zusätzliche Reinigungsmaßnahmen die Anforderung für Pharma- und Lebensmittelqualität erfüllt (Ullmann, Encyklopädie der technischen Chemie, 4. Auflage, Bd. 23, 709 (1983)).

Dazu kommt, daß das Produkt feinkristallin anfällt und stark staubt. Die Korngrößenverteilung reicht von 5 $\mu$ bis 150 $\mu$. Da Nicotinsäure sehr hautreizend wirkt (Kirk-Othmer, Encyclopedia of Chemical Technology, Vol. 24, Third Edition, 86) kann es zu arbeitshygienischen Problemen sowohl beim Hersteller als auch beim Weiterverarbeiter kommen.

Man versucht dieses Problem dadurch zu umgehen, daß man die feinkristalline Nicotinsäure granuliert und so ein Produkt mit einem Kornspektrum zwischen 100 und 315 $\mu$ (min 90 %) erhält (Lonza Revue 1/85, 14-15).

Aufgabe der Erfindung ist ein Verfahren, das es ermöglicht, ohne Granulations- und Reinigungsstufe, durch Verseifung von 3-Cyanopyridin mit Alkalihydroxid und anschließendes Ausfällen von Nicotinsäure mit hohen Ausbeuten eine grobkristalline, für Pharmazeutika und Lebensmittel geeignete Ware herzustellen.

Gegenstand der Erfindung ist ein Verfahren zur Herstellung grobkristalliner Nicotinsäure mit einer Reinheit, die der Pharma-Qualität (Europ. Arzneimittelbuch Bd. III, 164 (1978) entspricht, bei dem man 3-Cyanopyridin mit Alkalihydroxid verseift, und die Nicotinsäure anschließend aus dem gebildeten Nicotinat ausfällt, das dadurch gekennzeichnet ist, daß man eine wässrige Lösung mit einem Gehalt von 10 bis 50 Gew.-% 3-Cyanopyridin mit äquimolaren Mengen eines Alkalihydroxids, gelöst in Wasser, bei Temperaturen zwischen 130 und 190 °C und Drücken zwischen 1,5 und 2,0 MPa umsetzt, das bei der Reaktion freigesetzte Ammoniak abtrennt, die erhaltene ammoniakfreie Alkalinicotinatsäurelösung bei Temperaturen zwischen 99 und 135 °C innerhalb von 2 bis 4 h mit einer Mineralsäure kontinuierlich in der Weise versetzt, daß der pH-Wert der Lösung oberhalb von 3,7 bleibt, die sich bildende Nicotinsäuresuspension anschließend auf 0 bis 20 °C abkühlt, die auskristallisierte Nicotinsäure abtrennt, beispielsweise durch Zentrifugieren, wäscht, trocknet, bei Bedarf klassiert und das abgetrennte Überkorn vermahlt und das Unterkorn recycliert. Das erfindungsgemäße Verfahren ist sowohl kontinuierlich als auch diskontinuierlich durchführbar.

Man erhält ein Produkt, das staubfrei ist und Pharma-Qualität (Europ. Arzneimittelbuch Bd. III, 164 (1978) besitzt.

Die Kornverteilung ist abhängig von der Fällungstemperatur und der Zugabegeschwindigkeit der Mineralsäure zur Nicotinatlösung.

Das gewünschte Kornspektrum zwischen 150 und 300 $\mu$ (> 75%), erreicht man, wenn man die Mineralsäure innerhalb von 2 bis 4 h, bevorzugt 2,5 bis 3,5 h, kontinuierlich zusetzt.

Als Säure besonders geeignet sind Salzsäure und Schwefelsäure, Salzsäure in einer Konzentration von 10 bis 25 Gew.-%, bevorzugt 15 Gew.-%, Schwefelsäure, in einer Konzentration von 15 bis 25 Gew.-%, bevorzugt 20 Gew.-%.

Die Mineralsäuren dürfen nur in einer solchen Menge zudosiert werden, daß der pH-Wert der Lösung 3,7 nicht erreicht.

Besonders geeignet ist ein pH-Wert von 3,9 bis 4,1, insbesondere pH = 4,0.

Diese Tatsache überrascht den Fachmann, da nach allgemeinem Grundwissen Ampholyte -und dazu gehören die Pyridincarbonsäuren-im isoelektrischen Punkt die geringste Löslichkeit aufweisen. Dieser wird für Nicotinsäure mit pH = 3,42 angegeben (Beilstein, Monocarbonsäuren $C_nH_{2n-7}NO_2$ mit einem Stickstoffatom, E III/IV 22, Seite 350, 1979)

Als Alkalihydroxide besonders geeignet sind Natriumhydroxid und Kaliumhydroxid in Form von 10 bis 40 Gew.-%igen wässrigen Lösungen.

3-Cyanopyridin setzt man in Form von wässrigen Lösungen mit einem Gehalt von 10 bis 50 Gew.-% 3-Cyanopyridin ein.

Beide Reaktanden werden vorzugsweise vor der Einspeisung in den Hydrolysereaktor miteinander vermischt.

Die entstehende Nicotinatlösung fällt dann in der von den Ausgangslösungen abhängigen entsprechenden Verdünnung an.

Sie kann aber vor dem Ausfällen der Nicotinsäure auch durch bekannte Maßnahmen aufkonzentriert werden, insbesondere auf 30 bis 60 Gew.-%.

Wie aus der Zeichnung zu entnehmen, wird der Prozeß wie folgt geführt:
Über die Leitung 1 pumpt man eine wässrige 3-Cyanopyridinlösung, nach Zumischen von Alkalihydroxid (Leitung 2), in den beheizten Hydrolysereaktor 20. Die Reaktionsmischung gelangt über die Leitung 3 in den Stripper 21. Über die Leitung 4 entweicht das Ammoniak-Wasserdampfgemisch und wird einer Ammoniakrückgewinnung zugeführt. Durch die Leitung 5 gelangt die ammoniakfreie Alkalinicotinatlösung in den Fällreaktor 22, der gleichzeitig über die Leitung 6 mit wässriger Mineralsäure beschickt wird. Nach der Fällung zieht man die Nicotinsäuresuspension über die Leitung 7 ab und setzt die Kristallisation im Kristallisator 23 fort. Mit Hilfe der Leitung 8 beschickt man zur Entfeuchtung die Zentrifuge 24.

Danach gelangt der Filterkuchen über die Leitung 9 in den Trockner 25. Nach Klassierung 26 erhält man über die Leitung 11 die Nicotinsäure. Das Überkorn wird vermahlen und das Unterkorn recycliert.

In den folgenden Beispielen ist die Erfindung weiter beschrieben:

## Beispiel 1

Stündlich speist man 10 kg einer 30 %igen wässrigen 3-Cyanopyridinlösung, der stündlich 3,85 kg einer 30 %igen Natronlauge zugemischt werden, in ein auf 180 °C beheiztes Strömungsrohr ein.

Nach einer Verweilzeit von 10 Minuten bei einem Druck von 2,0 MPa wird die Reaktionslösung auf Normaldruck entspannt und mit Hilfe eines Dünnschichtverdampfers das entstandene Ammoniak abgetrennt. Aus der so erhaltenen ammoniakfreien Natriumnicotinatlösung fällt man innerhalb von 3 h bei 110 °C, durch Zugabe einer 15 %igen wässrigen Salzsäure bis zu einem pH-Wert von 3,9 bis 4,0, die Nicotinsäure aus. Nach Abkühlung der Suspension auf 2 °C wird zentrifugiert, gewaschen, getrocknet und die erhaltene Nicotinsäure durch Versieben klassiert. Mit einer Ausbeute von 97,2 %, bezogen auf das eingesetzte 3-Cyanopyridin, wird eine Nicotinsäure in Pharmaqualität erhalten. Die anfallenden Waschlaugen werden recycliert. Die Kornverteilung liegt zwischen 150 und 300 μ (>90 %).

## Beispiel 2

Es wird wie in Beispiel 1 verfahren, setzt jedoch zur Freisetzung der Nicotinsäure 20 %ige Schwefelsäure ein und kühlt die Suspension auf 20 °C ab.
Die Ausbeute beträgt 94,8 %. Die Reinheit entspricht den Anforderungen den Pharmaköpen. Das Kornspektrum liegt zwischen 150 und 300 μ (>90 %).

## Beispiel 3

Es wird wie in Beispiel 1 verfahren, setzt jedoch statt Natronlauge stündlich 5,39 kg einer 30 %igen Kalilauge ein und führt die Fällung analog Beispiel 2 mit Schwefelsäure durch. Die Ausbeute beträgt 93,6 %. Die Reinheit entspricht den Pharmaköpen. Das Kornspektrum liegt zwischen 150 und 300 μ (>90 %).

## Patentansprüche

1. Verfahren zur Herstellung grobkristalliner Nicotinsäure mit einer Reinheit, die der Pharmaqualität (Europ. Arzneimittelbuch Bd. III, 164 (1978) entspricht, bei dem man 3-Cyanopyridin mit Alkalihydroxid verseift, und die Nicotinsäure anschließend aus dem gebildeten Nicotinat ausfällt, dadurch gekennzeichnet, daß man eine wässrige Lösung mit einem Gehalt von 10 bis 50 Gew.-% 3-Cyanopyridin mit äquimolaren Mengen eines Alkalihydroxids, gelöst in Wasser, bei Temperaturen zwischen 130 und 190 °C und Drücken zwischen 1,5 und 2,0 MPa umsetzt, das bei der Reaktion freigesetzte Ammoniak abtrennt, die erhaltene ammoniakfreie Alkalinicotinatlösung bei Temperaturen zwischen 99 und 135 °C innerhalb von 2 bis 4 h mit einer Mineralsäure kontinuierlich in der Weise versetzt, daß der pH-Wert der Lösung oberhalb von 3,7 bleibt, die sich bildende Nicotinsäuresuspension anschließend auf 0 bis 20 °C abkühlt, die auskristallisierte Nicotinsäure abtrennt, gegebenenfalls klassiert und das abgetrennte Überkorn vermahlt und das Unterkorn recycliert

2. Verfahren gemäß Anspruch 1,
dadurch gekennzeichnet, daß man eine 10 bis 25 Gew.-%ige Salzsäure verwendet

3. Verfahren gemäß Anspruch 1,
dadurch gekennzeichnet, daß man eine 15 bis 25 Gew.-%ige Schwefelsäure verwendet.

4. Verfahren gemaß den Ansprüchen 1 bis 3,
dadurch gekennzeichnet, daß man der Lösung Mineralsäure zusetzt, bis sich ein pH-Wert von 3,9 bis 4,1 einstellt.

## Claims

1. A process for the preparation of coarsely crystalline nicotinic acid of pharmaceutical purity (Europ. Arzneimittelbuch Volume III, 164 (1978)) in which 3-cyanopyridine is saponified with an alkali metal hydroxide and the nicotinic acid is then precipitated from the nicotinate formed, characterised in that an aqueous solution containing from 10 to 50% by weight of 3-cyanopyridine is reacted at temperatures from 130 to 190°C and pressures from 1.5 to 2.0 mPa with equimolar quantities of an alkali metal hydroxide dissolved in water, the ammonia liberated in the reaction is separated off, a mineral acid is continuously added to the resulting ammonia-free alkali metal nicotinate solution at temperatures of from 99 to 135°C within 2 to 4 hours in such a manner that the pH of the solution remains above 3.7, the nicotinic acid suspension which forms is subsequently cooled to 0 to 20°C and the nicotinic acid which crystallises is separated off and optionally classified and the oversized particles which are separated off are ground down and the undersized particles are recycled.

2. A process according to Claim 1, characterised in that a 10 to 25% by weight hydrochloric acid is used.

3. A process according to Claim 1, characterised in that a 15 to 25% by weight sulphuric acid is used.

4. A process according to Claims 1 to 3, characterised in that mineral acid is added to the solution until the pH is adjusted to 3.9 - 4.1.

## Revendications

1. Procédé de fabrication d'acide nicotinique en cristaux grossiers d'une pureté qui correspond à la qualité pharmaceutique (Europ. Arzneimittelbuch Bd. III, 164 (1978), dans lequel on saponifie de la 3-cyanopyridine par un hydroxyde alcalin et on précipite ensuite l'acide nicotinique à partir du nicotinate formé, caractérisé en ce qu'on fait réagir une solution aqueuse d'une teneur pondérale de 10 à 50 % en poids de 3-cyanopyridine avec des quantités équimolaires d'un hydroxyde alcalin dissous dans l'eau à des températures comprises entre 130 et 190°C et sous des pressions comprises entre 1,5 et 2,0 MPa, on sépare l'ammoniac libéré au cours de la réaction, on fait réagir la solution de nicotinate alcalin exempte d'ammoniac obtenue en continu à des températures comprises entre 99 et 135°C dans un intervalle de 2 à 4 heures avec un acide minéral en sorte que la valeur du pH de la solution reste supérieure à 3,7, on refroidit ensuite la suspension d'acide nicotinique formée à 0 à 20°C, on sépare l'acide nicotinique cristallisé, on procède éventuellement à un criblage, on broie les refus supérieurs et on recycle les refus inférieurs.

2. Procédé selon la revendication 1, caractérisé en ce que l'on utilise un acide chlorhydrique à une concentration de 10 à 25 % en poids.

3. Procédé selon la revendication 1, caractérisé en ce que l'on utilise un acide sulfurique à une concentration de 15 à 25 % en poids.

4. Procédé selon les revendications 1 à 3, caractérisé en ce que l'on ajoute l'acide minéral à la solution jusqu'à ce que l'on obtienne un pH de 3,9 à 4,1.